# EUROPÄISCHE PATENTSCHRIFT

(11) **EP 0 619 094 B1**
(45) Veröffentlichungstag und Bekanntmachung des Hinweises auf die Patenterteilung: **26.08.1998**
(21) Anmeldenummer: 93121049.6
(22) Anmeldetag: 29.12.1993
(51) Int. Cl.: A61B 5/0408

(54) **Körperelektrode**
Body electrode
Electrode appliquée à un corps

(30) Priorität: 06.04.1993 DE 4311354
(43) Veröffentlichungstag der Anmeldung: 12.10.1994
(73) Patentinhaber: KENDALL Medizinische Erzeugnisse GmbH, 93333 Neustadt/Donau (DE)
(72) Erfinder: Barth, Heinz-Günter, D-38820 Halberstadt (DE); Krüger, Rainer, D-38820 Halberstadt (DE)
(74) Vertreter: Lins, Edgar, Dipl.-Phys. Dr.jur.

(56) Entgegenhaltungen:
- GB-A- 1 519 782
- GB-A- 1 587 817
- US-A- 4 050 453
- US-A- 4 102 331
- US-A- 4 109 648
- US-A- 4 442 315

## Beschreibung

Die Erfindung betrifft eine Körperelektrode mit einer Trägerschicht, bestehend aus einem Elektrodenteil, auf das ein elektrisch leitender Sensor aufgebracht ist, und einem Anschlußteil, in das ein Ansatz des leitenden Sensors übergeht und mit einer die freie Fläche des Sensors abdeckenden, für den Hautkontakt vorgesehenen klebenden und leitenden Schicht.

Körperelektroden weisen in herkömmlicher Bauart eine leitende und klebende Schicht auf, die den Hautkontakt herstellt. In diese Schicht ragt regelmäßig ein metallischer Sensor, überwiegend aus Ag/AgCl, hinein. An den Sensor ist ein Anschlußstück angeschweißt, angelötet oder angeklebt, das mit einem Anschlußkabel verbunden ist. Die Sensoranordnung und die klebende und leitende Schicht ist mit einer meist formstabileren Abdeckschicht geschützt.

Von der Ludlow Corporation ist unter der Bezeichnung "Perfect Circuit" eine Körperelektrode vorgestellt worden, die in der Technik von gedruckten Schaltungen hergestellt ist. Auf einer glasklar durchsichtigen Trägerschicht ist der Sensor in Form einer gedruckten Schaltung aufgebracht. Die Trägerschicht weist einseitig einen sich nur geringfügig konisch verschmalernden Ansatz auf, auf den vollflächig eine Anschlußfläche einstückig mit dem Sensor als gedruckte Schaltung aufgebracht ist. Die Sensoranordnung ist ausschließlich der Anschlußfläche mit einer klebenden und leitenden Schicht abgedeckt. Die Gesamtanordnung ist auf eine Schutzfolie aufgeklebt und kann zum Gebrauch von dieser abgezogen werden. Die so hergestellte Elektrode weist eine minimale Bauhöhe auf und ist einfach herzustellen. In ihrem Gebrauch stellen sich jedoch Nachteile heraus. Diese bestehen insbesondere darin, daß die Kunststoffträgerschicht einen hohen Biegewiderstand aufweist und somit für die Herstellung eines elektrischen Kontaktes, beispielsweise mit einer Klemmverbindung, erforderliche Verbiegungen der Anschlußfläche auf das Elektrodenteil unmittelbar übertragen werden, wodurch es zu partiellen Ablösungen der leitenden und klebenden Schicht von der Haut kommen kann, so daß Artefakte auftreten. Die Klemmverbindung and der Anschlußfläche des Elektrodenteils schränkt die Anwendbarkeit der Elektrode ein, weil der Patient wegen der Klemmverbindung beispielsweise nicht oder nur unter großer Behinderung auf der Elektrode liegen kann.

Die US-A-4,442,315 offenbart eine Körperelektrode, die aus einem Elektrodenteil, auf das ein elektrisch leitender Sensor aufgebracht ist, und aus einem als Anschlußteil dienenden Leitungsteil besteht, das mehrere an den Sensor angeschlossene Leiter aufweist, die parallel übereinander geführt sind und voneinander isoliert und abgeschirmt sind. Bei der so ausgestalteten Körperelektrode muß das Leitungsteil aus einer Vielzahl von Schichten aufgebaut werden, so daß es einen großen Querschnitt aufweist und dadurch zwangsweise einen hohen Biegewiderstand besitzt. Erforderliche Verbiegungen bei der Herstellung des elektrischen Kontakts mit einer Klemmverbindung werden dann ebenfalls unmittelbar auf das Elektrodenteil übertragen, so daß die vorstehend beschriebenen Nachteile auch hier gelten.

Die GB-A- 1,519,782 beschreibt eine Elektrode, die als Isolierschicht für das Leitungsteil Kunststoff oder Papier aufweisen kann.

Die vorliegende Erfindung hat zum Ziel, eine Körperelektrode der eingangs erwähnten Art in ihrer Handhabbarkeit und Zuverlässigkeit zu verbessern.

Erfindungsgemäß ist die Körperelektrode der eingangs erwähnten Art dadurch gekennzeichnet, daß das Leitungsteil wenigstens einige Zentimeter lang und höchstens wenige Millimeter breit ist, daß das Leitungsteil dreischichtig aufgebaut ist, wobei die Anschlußleitung auf der der Trägerschicht abgewandten Seite mit einer isolierenden Schicht abgedeckt und so allseitig isoliert ist und daß das Leitungsteil eine solche Dimensionierung und Flexibilität aufweist, daß sich an seinem Ende wirkende Biege- und Drehmomente nicht merklich auf das Elektrodenteil übertragen.

Erfindungsgemäß ist die Körperelektrode so ausgebildet, daß eine Anschlußleitung einstückig in sie integriert ist. Dies hat zur Folge, daß erstmalig zwischen Sensor und Anschlußleitung überhaupt keine Kontaktstelle mehr erforderlich ist, weil Sensor und Anschlußleitung einstückig und im selben Verfahrensschritt gebildet sein können. Die Integration der Anschlußleitung in die Elektrode hat ferner den Vorteil, daß bereits aufgrund der Formgebung eine weitgehende mechanische Entkopplung zwischen dem Anschlußende und dem Elektrodenteil entsteht. Diese aufgrund der Formgebung bestehende mechanische Entkopplung wird noch dadurch verbessert, daß eine sehr flexible Trägerschicht verwendet wird, die dafür sorgt, daß sich am Ende des Leitungsteils auftretende Biege- und Drehmomente nicht merklich auf das Elektrodenteil übertragen. Da die Kontaktierung zwischen der Anschlußleitung und beispielsweise einer mehrpoligen Weiche aufgrund der Anschlußleitung patientenfern erfolgt, entfällt eine Störung des Patienten durch das Verbindungselement, beispielsweise Klemmelement, mit dem die Kontaktierung hergestellt wird.

Trägerschicht und abdeckende Schicht können vorzugsweise aus demselben Material, z.B. Kunststoff gebildet sein. Die durchsichtige, vorzugsweise glasklare, Ausbildung der Schichten hat den Vorteil, daß die gedruckte Leitung bzw. der gedruckte Sensor optisch prüfbar sind, so daß etwaige Leitungsbrüche regelmäßig optisch erkennbar sind. Ferner kann die Haut unter der Elektrode, zumindest außerhalb des aufgedruckten Sensors und der aufgedruckten Leitung beobachtet werden, während die Elektrode aufgesetzt ist.

Zur Herstellung einer elektrischen Verbindung am Ende des Leitungsteils endet zweckmäßigerweise die abdeckende Schicht zur Ausbildung einer Kontaktfläche mit Abstand von dem freien Ende der Trägerschicht und der Anschlußleitung. Es ist denkbar, an das freie Ende der Anschlußleitung ein Kabel beispielsweise mit einer Klemmvorrichtung anzuschließen. Dabei ist besonders bevorzugt, eine mehradrige Weiche mit einer entsprechenden Anzahl von Klemmvorrichtungen auszubilden und so mehrere Elektroden mit ihren Anschlußleitungen an der Weiche zu kontaktieren. Dabei ist es zweckmäßig, wenn das Kontaktende der Anschlußleitung verbreitert ist, um ein versehentliches Einstecken in etwaige spannungsführende Teile unmöglich zu machen. Diese Art der Kontaktierung erscheint vorteilhaft gegenüber der ebenfalls denkbaren Kontaktierung des freien Endes der Anschlußleitung mit einem herkömmlichen Steckkontakt.

Die abdeckende Schicht kann sich vorteilhaft bis in den Elektrodenteil hinein erstrecken und sich dort verbreitern. Hierdurch wird die mechanische Stabilität der Verbindung der Anschlußleitung mit dem Elektrodenteil, insbesondere im Hinblick auf Auszugskräfte, wesentlich verbessert.

Eine optimale Form des Sensors für die erfindungsgemäße Elektrode besteht aus einem Kreisring mit mehreren radialen, sich zentrisch treffenden Stegen. Bei dieser Ausbildung kann sich die abdeckende Schicht bis in den Bereich des äußeren Randes des Kreisringes erstrecken.

In einer bevorzugten Ausführungsform weist das Leitungsteil ein Verhältnis von Länge zu Breite von > 50:1, vorzugsweise > 90:1 auf. In konkreten Ausführungsbeispielen beträgt die Breite des Leitungsteils etwa 3 mm bzw. 2 mm. Die Breite der aufgedruckten Anschlußleitung beträgt 2 mm bzw. 1 mm, so daß beiderseits der Anschlußleitung ein Streifen von etwa 0,5 mm existiert, auf dem die Trägerschicht unmittelbar mit der abdeckenden Schicht, flächig verbunden ist, so daß sich eine allseitige Isolierung der Anschlußleitung ergibt. Die Länge der Anschlußleitung ergibt sich aus dem jeweiligen Anwendungszweck und sollte regelmäßig > 20 cm sein. In der Praxis werden Längen von 23 bis 60 cm vorgesehen.

Die erfindungsgemäße Körperelektrode läßt sich mit einer Trägerschicht und einer abdeckenden Schicht aus Kunststoff ohne Probleme herstellen. Eine bevorzugte Alternative besteht jedoch darin, Papier als Trägerschicht zu verwenden und ggfs. die abdeckende Schicht durch einen aufgebrachten Isolierlack zu realisieren.

Im Unterschied zu der Technik von gedruckten Leitungen wird bei der erfindungsgemäßen Köperelektrode der Sensor und die Anschlußleitung vorzugsweise in einem echten Druckverfahren, insbesondere Siebdruckverfahren, auf die Trägerschicht aufgebracht. Sensor und Anschlußleitungen können dabei aus einem leitfähigen Lack bestehen, der vorzugsweise silberhaltig sein kann und vorzugsweise jedenfalls an seiner Oberfläche chloriert ist, um ein optimales Sensorsystem zu gewährleisten.

Der elektrisch leitende Sensor und die damit einstückig verbundene Anschlußleitung können in der erfindungsgemäßen Körperelektrode ohne weiteres aus röntgentransluzentem Material bestehen. Verwendung finden kann hierfür beispielsweise ein elektrisch leitender Lack auf der Basis einer Kohlenstoffüllung oder auf der Basis von Metallpulver, das die Röntgentransluzenz praktisch nicht merklich herabsetzt. Aufgrund der flexiblen und flachen Ausbildung der erfindungsgemäßen Körperelektrode eignet sich diese insbesondere auch zur Langzeitüberprüfung des Patienten und zur Durchführung von Röntgenaufnahmen.

Die Erfindung wird im folgenden anhand eines in der Zeichnung dargestellten Ausführungsbeispiels näher erläutert. Es zeigen:
- Figur 1 -: eine Draufsicht auf die durchsichtige Trägerschicht der erfindungsgemäßen Elektrode
- Figur 2 -: einen Längsschnitt durch die Elektrode gemäß Figur 1 mit der Andeutung eines elektrischen Anschlusses mit Hilfe einer Klemmverbindung
- Figur 3 -: einen Längsschnitt durch ein Ende des Leitungsteils mit einem angekrimpten Steckerstück.

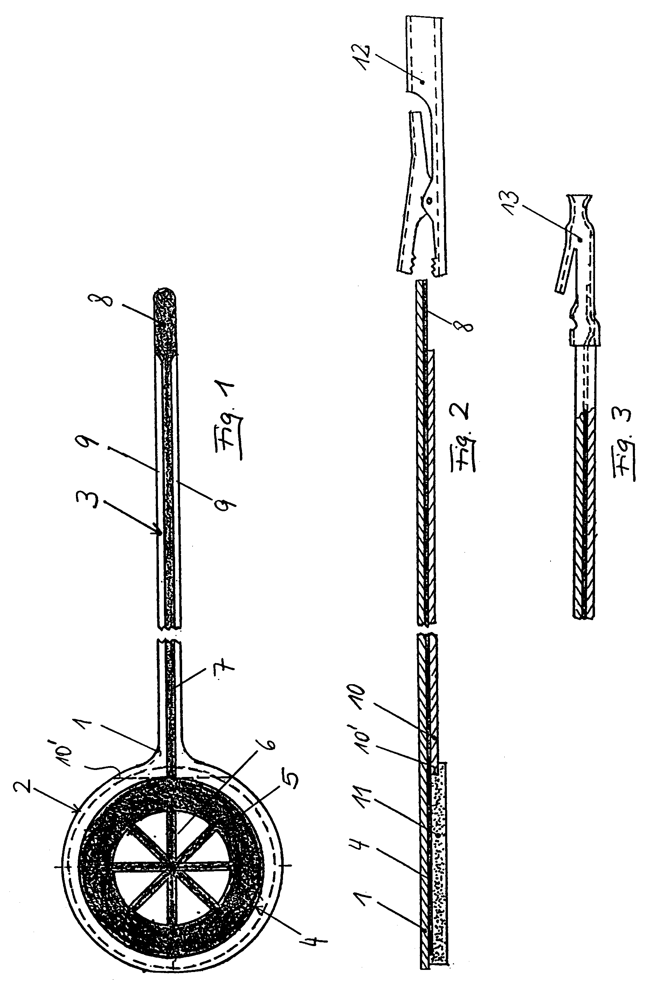

Figur 1 läßt eine Trägerschicht 1 erkennen, die aus einem Elektrodenteil 2 und einem Leitungsteil 3 besteht. Das Elektrodenteil 2 ist im wesentlichen kreisförmig ausgebildet, das Leitungsteil 3 länglich mit einer wesentlich größeren Länge als Breite. Auf das Elektrodenteil 2 ist ein Sensor 4 im Siebdruckverfahren aufgedruckt, der aus einem Kreisring 5 und acht speichenähnlichen Stegen 6 besteht, die radial ausgerichtet sind und sich im Zentrum des Kreisrings 5 treffen.

Mit dem Sensor 4 ist einstückig eine Anschlußleitung 7 verbunden, die sich über die volle Länge des Leitungsteils 3 der Kunststoffträgerschicht 1 erstreckt. Am freien Ende des Leitungsteils 3 ist die Anschlußleitung 7 verbreitert zur Ausbildung einer Kontaktfläche 8 dargestellt, die sich über die gesamte Breite des Leitungsteils erstreckt. Es ist erkennbar, daß sich beidseitig der gedruckten Anschlußleitung 7 Streifen 9 befinden, die frei von der gedruckten Anschlußleitung 7 sind. Über diese Streifen 9 besteht eine flächige Verbindung der Trägerschicht 1 mit einer auf der anderen Seite der aufgedruckten Anschlußleitung 7 aufgebrachten isolierenden Schicht 10 (Figur 2), so daß die gedruckte Anschlußleitung 7 allseitig isoliert ist. Die abdeckende isolierende Schicht 10 erstreckt sich in den Elektrodenteil 2 der Trägerschicht 1 hinein und endet mit einer vorderen Kante 10' im Bereich des äußeren Randes des Ringes 5 des Sensors 4. Im Bereich des Elektrodenteils 2 ist die abdeckende Schicht 10 entsprechend der Form der Trägerschicht 2 verbreitert, wodurch an dem Leitungsteil 3 wirkende Auszugskräfte verbessert aufgenommen werden.

Im Bereich des Elektrodenteils 2 ist der Sensor 4 auf seiner der Trägerschicht 1 abgewandten Seite durch eine klebende und leitende Schicht 11 abgedeckt. Diese haftet an der Trägerschicht 1 und an der metallischen Schicht des Sensors 4. Zur Sicherstellung eines Schutzes vor Kurzschlüssen erstreckt sich die leitende und klebende Schicht 11 bis über den Rand 10' der isolierenden Schicht 10.

Am freien Ende des Leitungsteils 3 endet die isolierende Schicht 10 mit Abstand von dem Ende der Trägerschicht 1 und der gedruckten Anschlußleitung 7, um die Kontaktfläche 8 einseitig frei zu lassen. Ein elektrischer Kontakt zu einem weiterführenden Kabel kann, wie Figur 2 zeigt, mit Hilfe einer Klemmverbindung 12 hergestellt werden, die vorzugsweise Bestnadteil einer vieladrigen Weiche zur Kontaktierung eine Mehrzahl von Anschlußleitungen ist.

In der bevorzugten in Figur 3 dargestellten Ausführungsform, wird über die Kontaktfläche 8 eine Verbindung mit einer angekrimpten Steckerbuchse 13 hergestellt, die direkt auf Steckerpins einer vieladrigen Kupplung aufschiebbar ist.

## Patentansprüche

1. Körperelektrode mit einer Trägerschicht (1) bestehend aus einem Elektrodenteil (2), auf das ein elektrisch leitender Sensor (4) aufgebracht ist, und einem als Anschlußteil dienenden Leitungsteil (3), auf das eine an den Sensor (4) angeschlossene Anschlußleitung (7) aufgebracht ist, und mit einer die freie Fläche des Sensors (4) abdeckenden, für den Hautkontakt vorgesehenen klebenden und leitenden Schicht (11), **dadurch gekennzeichnet, daß** das Leitungsteil (3) wenigstens einige Zentimeter lang und höchstens wenige Millimeter breit ist, daß das Leitungsteil (3) dreischichtig aufgebaut ist, wobei die Anschlußleitung (7) auf der der Trägerschicht (1) abgewandten Seite mit einer isolierenden Schicht (10) abgedeckt und so allseitig isoliert ist und daß das Leitungsteil (3) eine solche Dimensionierung und Flexibilität aufweist, daß sich an seinem Ende wirkende Biege- und Drehmomente nicht merklich auf das Elektrodenteil (2) übertragen.

2. Körperelektrode nach Anspruch 1, dadurch gekennzeichnet, daß die abdeckende Schicht (10) mit der Trägerschicht (1) beiderseits der Anschlußleitung (7) vollflächig verbunden ist.

3. Körperelektrode nach Anspruch 1 oder 2, dadurch gekennzeichnet, daß die abdeckende Schicht (10) und/oder die Trägerschicht (1) durchsichtig ist.

4. Körperelektrode nach einem der Ansprüche 1 bis 3, dadurch gekennzeichnet, daß die abdeckende Schicht (10) aus dem gleichen Material gebildet ist, wie die Trägerschicht (1).

5. Körperelektrode nach einem der Ansprüche 1 bis 4, dadurch gekennzeichnet, daß Anschlußleitung (7) und Sensor (4) einstückig hergestellt sind.

6. Körperelektrode nach einem der Ansprüche 1 bis 5, dadurch gekennzeichnet, daß die abdeckende Schicht (10) zur Ausbildung einer Kontaktfläche (8) mit Abstand von dem freien Ende der Trägerschicht (1) und der Anschlußleitung (7) endet.

7. Körperelektrode nach einem der Ansprüche 1 bis 6, dadurch gekennzeichnet, daß sich die abdeckende Schicht (10) bis in den Elektrodenteil (2) hinein erstreckt und sich dort verbreitert.

8. Körperelektrode nach einem der Ansprüche 1 bis 7, dadurch gekennzeichnet, daß der Sensor (4) in Form eines Kreisringes (5) mit mehreren radialen, sich zentrisch treffenden Stegen (6) ausgebildet ist.

9. Körperelektrode nach Anspruch 7 und 8, dadurch gekennzeichnet, daß sich die abdeckende Schicht (10) bis in den Bereich des äußeren Randes des Kreisringes (5) erstreckt.

10. Körperelektrode nach einem der Ansprüche 1 bis 9, dadurch gekennzeichnet, daß das Verhältnis von Länge zu Breite des Leitungsteils (3) > 50:1, vorzugsweise > 90:1 ist.

11. Körperelektrode nach einem der Ansprüche 1 bis 9, dadurch gekennzeichnet, daß das Leitungsteil (3) eine Breite von maximal 5 mm und eine Länge von wenigstens 20 cm aufweist.

12. Körperelektrode nach einem der Ansprüche 1 bis 11, dadurch gekennzeichnet, daß die Trägerschicht (1) und/oder die abdeckende Schicht (10) aus Kunststoff bestehen.

13. Körperelektrode nach einem der Ansprüche 1 bis 11, dadurch gekennzeichnet, daß die Trägerschicht (1) aus Papier gebildet ist.

14. Körperelektrode nach einem der Ansprüche 1 bis 13, dadurch gekennzeichnet, daß die abdeckende Schicht (10) aus einem Isolierlack besteht.

15. Körperelektrode nach einem der Ansprüche 1 bis 14, dadurch gekennzeichnet, daß der Sensor (4) und die Anschlußleitung (7) mit einem Siebdruckauftrag auf die Trägerschicht (1) aufgebracht sind.

16. Körperelektrode nach Anspruch 15, dadurch gekennzeichnet, daß der Sensor (4) und die Anschlußleitung (7) aus einem leitfähigen Lack bestehen.

17. Körperelektrode nach Anspruch 16, dadurch gekennzeichnet, daß der leitfähige Lack silberhaltig und vorzugsweise an seiner Oberfläche chloriert ist.

18. Körperelektrode nach einem der Ansprüche 1 bis 17, dadurch gekennzeichnet, daß sie aus röntgentransluzentem Material besteht.

## Claims

1. A body electrode comprising a carrier layer (1) consisting of an electrode part (2), to which an electrically conductive sensor (4) is applied, and a conduction part (3) which serves as connecting part and to which a connection line (7) connected to the sensor (4) is applied, and comprising an adhesive and conductive layer (11) which covers the free surface of the sensor (4) and is provided for the skin contact, characterized in that the conduction part (3) has a minimum length of a few centimetres and a maximum width of a few millimetres, that the conduction part (3) is constructed from three layers, the connection line (7) being covered with an insulating layer (10) on the side facing away from the carrier layer (1) and thus being insulated on all sides, and that the dimensioning and flexibility of the conduction part (3) are such that bending- and torsion moments active at one end are not noticeably transferred to the electrode part (2).

2. A body electrode according to Claim 1, characterised in that the entire surface of the covering layer (10) is connected to the carrier layer (1) on both sides of the connection line (7).

3. A body electrode according to Claim 1 or 2, characterised in that the covering layer (10) and/or the carrier layer (1) is/are transparent.

4. A body electrode according to one of Claims 1 to 3, characterised in that the covering layer (10) is formed from the same material as the carrier layer (1).

5. A body electrode according to one of Claims 1 to 4, characterised in that connection line (7) and sensor (4) are produced in one piece.

6. A body electrode according to one of Claims 1 to 5, characterised in that for the formation of a contact surface (8) the covering layer (10) terminates at a distance from the free end of the carrier layer (1) and the connection line (7).

7. A body electrode according to one of Claims 1 to 6, characterised in that the covering layer (10) extends into the electrode part (2) and widens therein.

8. A body electrode according to one of Claims 1 to 7, characterised in that the sensor (4) has the form of a circular ring (5) with a plurality of radial, centrally converging webs (6).

9. A body electrode according to Claim 7 and 8, characterised in that the covering layer (10) extends into the region of the outer edge of the circular ring (5).

10. A body electrode according to one of Claims 1 to 9, characterised in that the ratio of length to width of the conduction part (3) is > 50:1, preferably > 90:1.

11. A body electrode according to one of Claims 1 to 9, characterised in that the conduction part (3) has a maximum width of 5 mm and a minimum length of 20 cm.

12. A body electrode according to one of Claims 1 to 11, characterised in that the carrier layer (1) and/or the covering layer (10) consist of plastics material.

13. A body electrode according to one of Claims 1 to 11, characterised in that the carrier layer (1) is formed from paper.

14. A body electrode according to one of Claims 1 to 13, characterised in that the covering layer (10) consists of an insulating lacquer.

15. A body electrode according to one of Claims 1 to 14, characterised in that the sensor (4) and the connection line (7) are applied to the carrier layer (1) by screen printing.

16. A body electrode according to Claim 15, characterised in that the sensor (4) and the connection line (7) consist of a conductive lacquer.

17. A body electrode according to Claim 16, characterised in that the conductive lacquer contains silver and is preferably chlorinated at its surface.

18. A body electrode according to one of Claims 1 to 17, characterised in that it consists of X-ray-translucent material.

## Revendications

1. Electrode pour le corps, comportant une couche porteuse (1) constituée d'une partie électrode (2) sur laquelle est installé un capteur électriquement conducteur (4), et d'une partie conductrice (3) servant de partie de raccordement, sur laquelle est installée une ligne de raccordement (7) raccordée au capteur (4), et comportant une couche conductrice et collante (11) prévue pour le contact avec la peau, recouvrant la surface libre du capteur (4), caractérisée en ce que la partie conductrice (3) est longue d'au moins quelques centimètres et large d'au plus un petit nombre de millimètres, en ce que la partie conductrice (3) est constituée à trois couches, la ligne de raccordement (7) étant recouverte d'une couche isolante (10) du côté opposé à la couche porteuse (1) et étant ainsi isolée de tous côtés, et en ce que la partie conductrice (3) présente un dimensionnement et une flexibilité telles que les moments de rotation et de flexion s'exerçant à son extrémité ne sont pas notablement transmis à la partie électrode (2).

2. Electrode pour le corps selon la revendication 1, caractérisée en ce que la couche de recouvrement (10) est reliée par toute la surface avec la couche porteuse (1) des deux côtés de la ligne de raccordement (7).

3. Electrode pour le corps selon la revendication 1 ou 2, caractérisée en ce que la couche de recouvrement (10) et/ou la couche porteuse (1) est transparente.

4. Electrode pour le corps selon l'une des revendications 1 a 3, caractérisée en ce que la couche de recouvrement (10) est constituée de la même matière que la couche porteuse (1).

5. Electrode pour le corps selon l'une des revendications 1 à 4, caractérisée en ce que la ligne de raccordement (7) et le capteur (4) sont fabriqués d'une seule pièce.

6. Electrode pour le corps selon l'une des revendications 1 à 5, caractérisée en ce que pour la réalisation d'une face de contact (8), la couche de recouvrement (10) se termine à distance de l'extrémité libre de la couche porteuse (1) et de la ligne de raccordement (7).

7. Electrode pour le corps selon l'une des revendications 1 à 6, caractérisée en ce que la couche de recouvrement (10) s'étend jusque dans la partie électrode (2) et s'y élargit.

8. Electrode pour le corps selon l'une des revendications 1 à 7, caractérisée en ce que le capteur (4) est réalisé sous la forme d'un anneau circulaire (5) avec plusieurs barrettes radiales (6) se rejoignant au centre.

9. Electrode pour le corps selon les revendications 7 et 8, caractérisée en ce que la couche de recouvrement (10) s'étend jusque dans la zone du bord extérieur de l'anneau circulaire (5).

10. Electrode pour le corps selon l'une des revendications 1 à 9, caractérisée en ce que le rapport entre la longueur et la largeur de la partie conductrice (3) est supérieur à 50:1, de préférence supérieur à 90:1.

11. Electrode pour le corps selon l'une des revendications 1 à 9, caractérisée en ce que la partie conductrice (3) présente une largeur d'au maximum 5 mm et une longueur d'au minimum 20 cm.

12. Electrode pour le corps selon l'une des revendications 1 à 11, caractérisée en ce que la couche porteuse (1) et/ou la couche de recouvrement (10) sont constituées de matière plastique.

13. Electrode pour le corps selon l'une des revendications 1 à 11, caractérisée en ce que la couche porteuse (1) est réalisée en papier.

14. Electrode pour le corps selon l'une des revendications 1 à 13, caractérisée en ce que la couche de recouvrement (10) consiste en un vernis isolant.

15. Electrode pour le corps selon l'une des revendications 1 à 14, caractérisée en ce que le capteur (4) et la ligne de raccordement (7) sont installées par un dépôt sérigraphique sur la couche porteuse (1).

16. Electrode pour le corps selon la revendication 15, caractérisée en ce que le capteur (4) et la ligne de raccordement (7) consistent en un vernis conducteur.

17. Electrode pour le corps selon la revendication 16, caractérisée en ce que le vernis conducteur contient de l'argent et est de préférence chloré sur sa surface.

18. Electrode pour le corps selon l'une des revendications 1 à 17, caractérisée en ce qu'elle est constituée de matière translucide pour les rayons X.
